# EUROPEAN PATENT SPECIFICATION

(11) **EP 3 939 976 B1**
(45) Date of publication and mention of the grant of the patent: **01.05.2024**
(21) Application number: 20791861.6
(22) Date of filing: 17.04.2020
(51) Int. Cl.: C07D 413/14, A61K 31/4725, A61P 19/00

(54) **PRODRUG OF CASPASE INHIBITOR**
PRODRUG EINES CASPASE-INHIBITORS
PROMÉDICAMENT D'INHIBITEUR DE CASPASE

(30) Priority: 19.04.2019 KR 20190046027
(43) Date of publication of application: 19.01.2022
(73) Proprietor: LG CHEM, LTD., Yeongdeungpo-gu, Seoul 07336 (KR)
(72) Inventor: CHOI, Sei Hyun, Seoul 07796 (KR); SONG, Jeong Uk, Seoul 07796 (KR); PARK, Ah Byeol, Seoul 07796 (KR); KIM, Jung Joon, Seoul 07796 (KR); PARK, Hyun Seo, Seoul 07796 (KR)
(74) Representative: Hoffmann Eitle
(86) International application number: PCT/KR2020/005134
(87) International publication number: WO 2020/213970

(56) References cited:
- WO-A1-2005/021516
- WO-A1-2010/005765
- WO-A1-2017/059427
- WO-A2-2007/015931
- WO-A2-2014/048940
- KR-A- 20060 094 868
- US-A1- 2010 120 843

## Description

### TECHNICAL FIELD

The present invention relates to an isoxazoline derivative having lactone moiety as a prodrug of caspase inhibitor and a pharmaceutical composition comprising the same.

### BACKGROUND ART

Caspases are a type of enzymes and are cysteine proteases that exist as an α2β2 tetramer. Caspase inhibitors interfere with the activity of these caspases, thereby regulating inflammation or apoptosis caused by the action of caspases. Diseases in which symptoms can be eliminated or alleviated by administration of these compounds include osteoarthritis, rheumatoid arthritis, degenerative arthritis, destructive bone disorder, hepatic diseases caused by hepatitis virus, acute hepatitis, hepatocirrhosis, brain damages caused by hepatitis virus, human fulminant liver failure, sepsis, organ transplantation rejection, ischemic cardiac disease, dementia, stroke, brain impairment due to AIDS, diabetes, gastric ulcer, etc.

Among compounds having various structures known as caspase inhibitors, isoxazoline derivatives were filed as Korean Patent Application Nos. 10-2004-0066726, 10-2006-0013107 and 10-2008-0025123. In addition, a prodrug of a caspase inhibitor based on an isoxazoline derivative was disclosed in International Publication No. WO 2007/015931 (Applicant: Vertex Pharmaceuticals Incorporated, USA). KR 2006-0094868 A also discloses isooxazoline derivatives used as caspase inhibitors and pharmaceutical compositions comprising the same.

### DISCLOSURE OF INVENTION

### TECHNICAL PROBLEM

The present invention is intended to improve bioavailability by developing a prodrug of an isoxazoline derivative having the structure of Formula 2 which is an effective inhibitor against caspase. In addition, the caspase inhibitor of Formula 2 has high solubility in water and high hydrophilicity, so it may be advantageous for the development of oral formulations, but there may be a disadvantage in the development of long-acting formulations. As such, the present invention is intended to develop a prodrug form of the caspase inhibitor of Formula 2 having hydrophobicity to be advantageous for long-acting formulations.

### SOLUTION TO PROBLEM

In order to achieve the above object, the present invention provides a compound of the following Formula 1, or a pharmaceutically acceptable salt or isomer thereof: 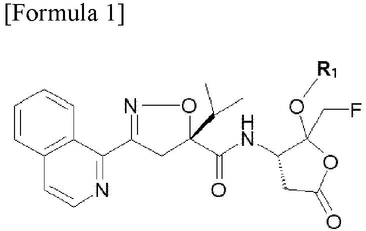 wherein
R₁ represents alkyl, cycloalkyl, aryl or -C(O)R₂;
R₂ represents alkyl, cycloalkyl, aryl, arylalkyl, or heteroaryl including one or more heteroatoms selected from N, O and S; and
the alkyl, cycloalkyl, arylalkyl and heteroaryl are optionally substituted, and the substituent may be one or more selected from alkyl, cycloalkyl, hydroxy, halo, haloalkyl, acyl, amino, alkoxy, carboalkoxy, carboxy, carboxyamino, cyano, nitro, thiol, aryloxy, sulfoxy and guanido group.

The compound of Formula 1 according to the present invention may form a pharmaceutically acceptable salt. A pharmaceutically acceptable salt may include an acid-addition salt which is formed from an inorganic acid such as hydrochloric acid, sulfuric acid, nitric acid, phosphoric acid, hydrobromic acid and hydroiodic acid; an organic acid such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acid such as methanesulfonic acid, ethanesulfonic acid, benzenesulfonic acid and p-toluenesulfonic acid, which form non-toxic acid-addition salt including pharmaceutically acceptable anion. In addition, a pharmaceutically acceptable carboxylic acid salt includes the salt with alkali metal or alkali earth metal such as lithium, sodium, potassium, calcium and magnesium; salts with amino acid such as lysine, arginine and guanidine; an organic salt such as dicyclohexylamine, N-methyl-D-glucamine, tris(hydroxymethyl)methylamine, diethanolamine, choline and triethylamine. The compound of Formula 1 according to the present invention may be converted into their salts by conventional methods.

Meanwhile, since the compound of Formula 1 according to the present invention can have an asymmetric carbon center and asymmetric axis or plane, they can exist as E- or Z-isomer, R- or S-isomer, racemic mixtures or diastereoisomer mixtures and each diastereoisomer, all of which are within the scope of the present invention.

Herein, unless indicated otherwise, the term "the compound of Formula 1" is used to mean all the compounds of Formula 1, including the pharmaceutically acceptable salts and isomers thereof.

Herein, the following concepts defining the substituents are used to define the compound of Formula 1.

The term "halogen" or "halo" means fluoride (F), chlorine (Cl), bromine (Br) or iodine (I).

The term "alkyl" means straight or branched hydrocarbons, may include a single bond, a double bond or a triple bond, and is preferably C₁-C₁₀ alkyl. Examples of alkyl include, but are not limited to, methyl, ethyl, *n*-propyl, *i*-propyl, *n*-butyl, *i*-butyl, *tert*-butyl, acetylene, vinyl, trifluoromethyl and the like.

The term "cycloalkyl" means partially or fully saturated single or fused ring hydrocarbons, and is preferably C₃-C₁₀-cycloalkyl. Examples of cycloalkyl include, but are not limited to, cyclopropyl, cyclobutyl, cyclopentyl, cyclohexyl, cycloheptyl and the like.

Unless otherwise defined, the term "alkoxy" means alkyloxy having 1 to 10 carbon atoms.

The term "aryl" means aromatic hydrocarbons, preferably C₅-C₁₂ aryl, more preferably C₆-C₁₀ aryl. Examples of aryl include, but are not limited to, phenyl, naphthyl and the like.

The term "heteroaryl" means 3- to 12-membered, more preferably 5- to 10-membered aromatic hydrocarbons which form a single or fused ring-which may be fused with benzo or C₃-C₈ cycloalkyl-including one or more heteroatoms selected from N, O and S as a ring member. Examples of heteroaryl include, but are not limited to, pyridinyl, pyrimidinyl, pyridazinyl, pyrazinyl, oxadiazolyl, isoxadiazolyl, tetrazolyl, triazolyl, indolyl, indazolyl, isoxazolyl, oxazolyl, thiazolyl, isothiazolyl, furanyl, benzofuranyl, imidazolyl, thiophenyl, benzthiazole, benzimidazole, quinolinyl, indolinyl, 1,2,3,4-tetrahydroisoquinolyl, 3,4-dihydroisoquinolinyl, thiazolopyridyl, 2,3-dihydrobenzofuran, 2,3-dihydrothiophene, 2,3-dihydroindole, benzo[1,3]dioxin, chroman, thiochroman, 1,2,3,4-tetrahydroquinoline, 4H-benzo[1,3]dioxin, 2,3-dihydrobenzo[1,4]-dioxin, 6,7-dihydro-5H-cyclopenta[d]pyrimidine and the like.

Aryl-alkyl, alkyl-aryl and heteroaryl-alkyl mean groups which are formed by the combination of the above-mentioned aryl and alkyl, or heteroaryl and alkyl Examples include, but are not limited to, benzyl, thiophenemethyl, pyrimidinemethyl and the like.

According to one embodiment of the present invention, in Formula 1 R₁ represents C₁-C₈ alkyl or -C(O)R₂; and R₂ represents C₁-C₂₀ alkyl, C₆-C₁₀ aryl or C₆-C₁₀ aryl-C₁-C₇ alkyl.

According to another embodiment of the present invention, in Formula 1 R₁ represents C₁-C₅ alkyl or -C(O)R₂; R₂ represents C₁-C₁₅ alkyl, C₆-C₁₀ aryl or C₆-C₁₀ aryl-C₁-C₅ alkyl; and the substituent is alkyl or haloalkyl.

Representative compounds of Formula 1 according to the present invention include, but are not limited to, the following compounds:
(2 S, 3 S)-2-(fluoromethyl)-3 -((R)-5-isopropyl-3-(isoquinolin-1-yl)-4,5-dihydroisoxazole-5-carboxamido)-5-oxotetrahydrofuran-2-yl acetate;
(2 S, 3 S)-2-(fluoromethyl)-3 -((R)-5-isopropyl-3 -(isoquinolin-1-yl)-4,5-dihydroisoxazole-5-carboxamido)-5-oxotetrahydrofuran-2-yl propionate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl isobutyrate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl pivalate;
(2 S, 3 S)-2-(fluoromethyl)-3-((R)-5-isopropyl-3 -(isoquinolin-1-yl)-4,5-dihydroisoxazole-5-carboxamido)-5-oxotetrahydrofuran-2-yl 3-methylbutanoate;
(2 S, 3 S)-2-(fluoromethyl)-3-((R)-5-isopropyl-3 -(isoquinolin-1-yl)-4,5-dihydroisoxazole-5-carboxamido)-5-oxotetrahydrofuran-2-yl 3,3-dimethylbutanoate;
(2S,3S)-2-(fluoromethyl)-3-((R)-5-isopropyl-3 -(isoquinolin-1-yl)-4,5-dihydroisoxazole-5-carboxamido)-5-oxotetrahydrofuran-2-yl palmitate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl benzoate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 4-(trifluoromethyl)benzoate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 2-phenylacetate;
(5R)-N-((3S)-2-ethoxy-2-(fluoromethyl)-5-oxotetrahydrofuran-3-yl)-5-isopropyl-3-(isoquinolin-1-yl)-4,5-dihydroisoxazole-5-carboxamide; and
(5R)-N-((3S)-2-(fluoromethyl)-2-methoxy-5-oxotetrahydrofuran-3-yl)-5-isopropyl-3-(isoquinolin-1-yl)-4,5-dihydroisoxazole-5-carboxamide.

The terms and abbreviations used herein retain their original meanings unless indicated otherwise.

The present invention also provides a method for preparing the compound of Formula 1. Hereinafter, the method for preparing the compound of Formula 1 is explained based on exemplary reactions in order to illustrate the present invention. However, a person skilled in the art could prepare the compound of Formula 1 by various methods based on the structure of Formula 1, and such methods should be interpreted as being within the scope of the present invention. That is, the compound of Formula 1 may be prepared by the methods described herein or by combining various methods disclosed in the prior art, which should be interpreted as being within the scope of the present invention. Accordingly, a method for preparing the compound of Formula 1 is not limited to the following methods.

The compound of Formula 1 of the present invention may be prepared from the compound of Formula 2 according to the method of the following Reaction Scheme 1. The compound of Formula 1-which is a prodrug-may be synthesized by the use of the compound of Formula 2 and an acyl chloride derivative, triethylamine (Et₃N), DMAP (4-dimethyl aminopyridine) and dichloromethane (DCM) solvent, or after synthesizing the intermediate by the use of the compound of Formula 2 and p-tosylic acid, trimethoxymethane, ethanol or methanol, and the compound synthesized by reacting lithium hydroxide, water and THF is used in trifluoroacetic acid and dichloromethane solvent.

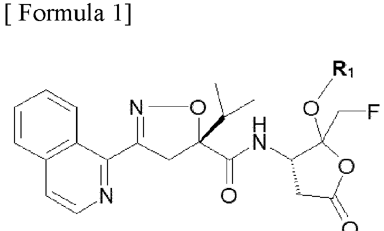

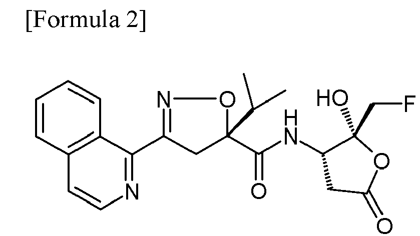

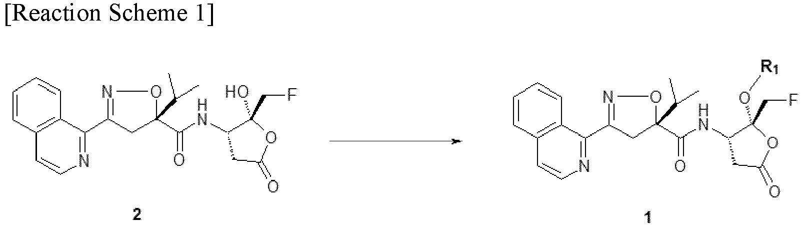

A compound not specifically described in the preparation method of the present specification is a known compound or a compound that can be easily synthesized from a known compound by a known synthesis method or a similar method.

The compound of Formula 1 obtained by the above methods can be separated or purified from the reaction products by conventional methods such as recrystallization, ionospheresis, silica gel column chromatography or ion-exchange chromatography.

As explained above, the compounds according to the present invention, starting materials or intermediates for the preparation thereof can be prepared by a variety of methods, which should be interpreted as being within the scope of the present invention in respect to the preparation of the compound of Formula 1.

The compound of Formula 1 according to the present invention can be used as a prodrug of caspase inhibitor. Accordingly, the present invention provides a pharmaceutical composition for use in the prevention or treatment of inflammation or apoptosis comprising the compound of Formula 1, or a pharmaceutically acceptable salt or isomer thereof as an active ingredient, together with a pharmaceutically acceptable carrier.

Exemplary diseases that can be prevented or treated by the pharmaceutical composition according to the present invention include, but are not limited to, those selected from the group consisting of apoptosis-associated diseases, inflammatory diseases, osteoarthritis, rheumatoid arthritis, degenerative arthritis and destructive bone disorders.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethyl sulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the compound of Formula 1 or a pharmaceutically acceptable salt or isomer thereof-is mixed with selected pharmaceutically acceptable carriers considering the dosage form to be prepared. For example, the pharmaceutical composition of the present invention can be formulated as injections, oral preparations and the like, as needed.

The pharmaceutical composition of the present invention may be formulated in oral dosage form, injection form or patch form, but may not be limited thereto.

The compound of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The compound of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the compounds of the present invention with at least one carrier selected from inert diluents such as sucrose, lactose or starch, lubricants such as magnesium stearate, disintegrating agents, binders and the like.

In the case of parenteral formulations, sterilized water is used usually and other ingredient(s) such as a dissolution adjuvant may also be comprised. Injection formulations, for example, sterilized aqueous- or oil-based suspension for injection may be prepared according to known techniques by using appropriate dispersing agent, wetting agent or suspending agent. The solvents useful for this purpose include water, ringer solution and isotonic NaCl solution, and sterilized, immobilized oils are also used as a solvent or a suspending medium conventionally. Any non-irritant immobilized oils including mono- and di-glycerides may be used for this purpose, and fatty acids such as an oleic acid may be used for an injection formulation. In the case of percutaneous formulations, a penetration-enhancing agent and/or a suitable wetting agent may be used as a carrier, optionally in combination with suitable non-irritant additive(s) to the skin. As such additives, those helpful in enhancing the administration through the skin and/or preparing the desired composition may be selected. The percutaneous formulation may be administered in various ways-for example, such as a transdermal patch, a spot-on treatment or an ointment.

The compound or pharmaceutical composition comprising the same according to the present invention can be administered in combination with other drugs-for example, other caspase inhibitors and/or caspase inhibitor prodrugs.

The dose of the compound of Formula 1 according to the present invention is determined by a physician's prescription considering the patient's body weight, age, and specific condition and seriousness of the disease. When the compound of the present invention is administered for clinical purposes, the total daily dose to be administered to the host in a single dose or in separate doses is preferably in the range of about 5 to 500 mg/kg of body weight, but the specific dose level for a specific patient may vary depending on the patient's weight, sex, health status, diet, drug administration time, administration method, excretion rate, drug mixture, disease severity, etc.

Herein, the term "treatment" is used to mean deterring, delaying or ameliorating the progress of diseases in a subject exhibiting symptoms of diseases.

### ADVANTAGEOUS EFFECTS OF INVENTION

The present invention relates to a novel compound having the structure of Formula 1, which is a prodrug of an isoxazoline derivative-which is a caspase inhibitor-having the structure of Formula 2. That is, the compound of Formula 1 acts as a prodrug of a caspase inhibitor. The prodrug compound having the structure of Formula 1 is converted into the active form of the caspase inhibitor of Formula 2 by an esterase isoenzyme in the body. These prodrug compounds have advantages over the caspase inhibitor of Formula 2 in terms of pharmacokinetics. Specifically, the prodrug compound of Formula 1 has increased drug durability compared to the caspase inhibitor of Formula 2. In addition, because the prodrug compound of Formula 1 can be converted into the caspase inhibitor of Formula 2 by a degrading enzyme in the human body and has hydrophobicity itself, it may be suitable for a long-acting formulation.

### BRIEF DESCRIPTION OF DRAWINGS

Figure 1 is a graph representing the results of the hydrolysis test of the compound of Formula 1 prepared according to the Example of the present invention.
Figure 2 is a graph representing the results of the pharmacokinetics test of the compounds of Formula 1 prepared according to the Examples of the present invention.

### MODE FOR THE INVENTION

Hereinafter, the present invention will be described in more detail through preparation examples and examples. However, these examples are only illustrative, and the scope of the present invention is not limited thereto.

### Example 1: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl acetate

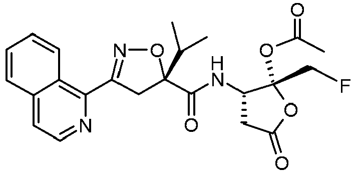

The compound of Formula 2 (5.0 g, 12.0 mmol) was dissolved in dichloromethane (50 mL), and then acetyl chloride (0.94 mL, 13.2 mmol, 1.1 equiv), triethylamine (2.52 mL, 18.0 mmol, 1.5 equiv) and 4-dimethylaminopyridine (0.15 g, 1.2 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (25 mL). After adding water (25 mL) and stirring, the organic layer was separated and distilled under reduced pressure. The obtained mixture was recrystallized in a 1:5 mixture of ethyl acetate and hexane (EtOAc:hexane=1:5) to obtain 3.0 g (yield: 54%) of the title compound.
¹H NMR (400 MHz, CDCl₃) δ 9.12 (d, 1H), 8.55 (d, 1H), 7.87 (d, 1H), 7.74-7.69 (m, 3H), 7.08 (d, 1H), 5.22 (m, 1H), 4.69 (d, 2H), 4.03 (d, 1H), 3.83 (d, 1H), 2.97 (m, 2H), 2.38 (m, 1H), 2.18 (s, 3H), 1.05 (dd, 6H)

### Example 2: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl propionate

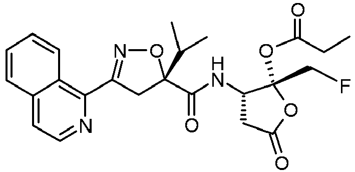

The compound of Formula 2 (1.0 g, 2.4 mmol) was dissolved in dichloromethane (20 mL), and then propionyl chloride (0.23 mL, 2.65 mmol, 1.1 equiv), triethylamine (0.5 mL, 3.61 mmol, 1.5 equiv) and 4-dimethylaminopyridine (0.03 g, 0.24 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was column separated by the use of a 1:2 mixture of ethyl acetate and hexane (EtOAc:hexane=1:2) to obtain 0.25 g (yield: 23%) of the title compound.
¹H NMR (400 MHz, CDCl₃) δ 9.12 (d, 1H), 8.55 (d, 1H), 7.87 (d, 1H), 7.74-7.69 (m, 3H), 7.08 (d, 1H), 5.22 (m, 1H), 4.69 (d, 2H), 4.03 (d, 1H), 3.83 (d, 1H), 2.98 (m, 2H), 2.45 (m, 2H), 2.37 (m, 1H), 1.20 (t, 3H), 1.05 (dd, 6H)

### Example 3: (2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl isobutyrate

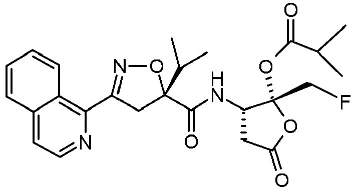

The compound of Formula 2 (1.0 g, 2.4 mmol) was dissolved in dichloromethane (20 mL), and then isobutyryl chloride (0.73 g, 2.65 mmol, 1.1 equiv), triethylamine (0.5 mL, 3.61 mmol, 1.5 equiv) and 4-dimethylaminopyridine (0.03 g, 0.24 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was column separated by the use of a 1:2 mixture of ethyl acetate and hexane (EtOAc:hexane=1:2) to obtain 0.06 g (yield: 5%) of the title compound.
¹H NMR (400 MHz, CDCl₃) δ 9.12 (d, 1H), 8.55 (d, 1H), 7.88 (d, 1H), 7.74-7.69 (m, 3H), 7.09 (d, 1H), 5.25 (m, 1H), 4.69 (d, 2H), 4.03 (d, 1H), 3.83 (d, 1H), 2.95 (m, 2H), 2.63 (m, 1H), 2.38 (m, 1H), 1.26 (dd, 6H), 1.05 (dd, 6H)

### Example 4: (2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl pivalate

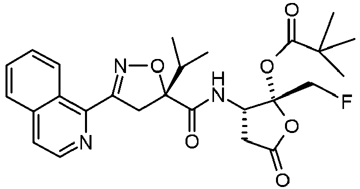

The compound of Formula 2 (0.5 g, 1.2 mmol) was dissolved in dichloromethane (20 mL), and then pivaloyl chloride (0.17 g, 1.4 mmol, 1.1 equiv) and 4-dimethylaminopyridine (0.29 g, 2.4 mmol, 2.0 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.1 g (yield: 17%) of the title compound.

¹H NMR (400 MHz, CDCl₃) δ 9.11 (d, 1H), 8.55 (d, 1H), 7.88 (d, 1H), 7.71 (m, 3H), 7.11 (d, 1H), 5.26 (m, 1H), 4.68 (d, 2H), 4.02 (d, 1H), 3.83 (d, 1H), 2.95 (m, 2H), 2.39 (m, 1H), 2.27 (s, 2H), 1.28 (s, 9H), 1.05 (dd, 6H)

### Example 5: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 3-methylbutanoate

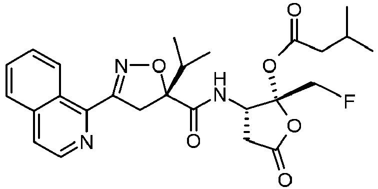

The compound of Formula 2 (0.5 g, 1.2 mmol) was dissolved in dichloromethane (20 mL), and then isovaleryl chloride (0.17 g, 1.4 mmol, 1.1 equiv), triethylamine (0.18 g, 1.8 mmol, 1.5 equiv) and 4-dimethylaminopyridine (0.015 g, 0.12 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.13 g (yield: 17%) of the title compound.
¹H NMR (400 MHz, CDCl₃) δ 9.11 (d, 1H), 8.52 (d, 1H), 7.86 (d, 1H), 7.71 (m, 3H), 7.11 (d, 1H), 5.23 (m, 1H), 4.71 (d, 2H), 4.04 (d, 1H), 3.83 (d, 1H), 2.95 (m, 2H), 2.35 (m, 1H), 2.28 (d, 2H), 2.15 (m, 1H), 1.05 (dd, 12H)

### Example 6: (2S,3S)-2-(fluoromethyl)-3-((R)-5-isopropyl-3-(isoquinolin-1-yl)-4,5-dihvdroisoxazole-5-carboxamido)-5-oxotetrahydrofuran-2-vl 3,3-dimethylbutanoate

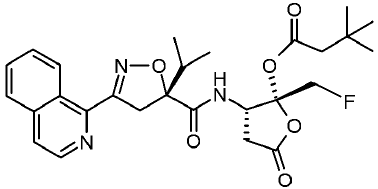

The compound of Formula 2 (0.5 g, 1.2 mmol) was dissolved in dichloromethane (20 mL), and then t-butyl acetyl chloride (0.19 g, 1.4 mmol, 1.1 equiv), triethylamine (0.18 g, 1.8 mmol, 1.5 equiv) and 4-dimethylaminopyridine (0.015 g, 0.12 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.14 g (yield: 23%) of the title compound.
¹H NMR (400 MHz, CDCl₃) δ 9.12 (d, 1H), 8.55 (d, 1H), 7.88 (d, 1H), 7.71 (m, 3H), 7.13 (d, 1H), 5.23 (m, 1H), 4.71 (d, 2H), 4.04 (d, 1H), 3.83 (d, 1H), 2.95 (m, 2H), 2.35 (m, 1H), 2.27 (s, 2H), 1.05 (dd, 15H)

### Example 7: (2S, 3S)-2-(fluoromethyl)-3-((R)-5-isopropyl-3-(isoquinolin-1-yl)-4,5-dihydroisoxazole-5-carboxamido)-5-oxotetrahydrofuran-2-yl palmitate

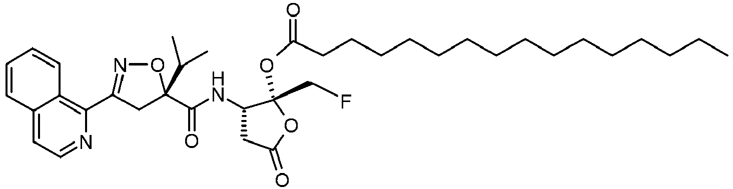

The compound of Formula 2 (1.0 g, 2.4 mmol) was dissolved in dichloromethane (20 mL), and then palmitoyl chloride (0.73 g, 2.65 mmol, 1.1 equiv), triethylamine (0.5 mL, 3.61 mmol, 1.5 equiv) and 4-dimethylaminopyridine (0.03 g, 0.24 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was column separated by the use of a 1:2 mixture of ethyl acetate and hexane (EtOAc:hexane=1:2) to obtain 0.11 g (yield: 7%) of the title compound.
¹H NMR (400 MHz, CDCl₃) δ 9.12 (d, 1H), 8.55 (d, 1H), 7.88 (d, 1H), 7.74-7.69 (m, 3H), 7.09 (d, 1H), 5.23 (m, 1H), 4.69 (d, 2H), 4.03 (d, 1H), 3.83 (d, 1H), 2.95 (m, 2H), 2.41 (m, 2H), 2.38 (m, 1H), 1.68 (m, 2H), 1.35-1.24 (m, 24H), 1.05 (dd, 6H), 0.88 (t, 3H)

### Example 8: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl benzoate

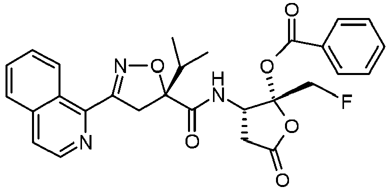

The compound of Formula 2 (0.5 g, 1.2 mmol) was dissolved in dichloromethane (20 mL), and then benzoyl chloride (0.17 g, 1.4 mmol, 1.1 equiv), triethylamine (0.18 g, 1.8 mmol, 1.5 equiv) and 4-dimethylaminopyridine (0.15 g, 1.2 mmol, 1.0 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.14 g (yield: 22%) of the title compound.
¹H NMR (400 MHz, CDCl₃) δ 9.13 (d, 1H), 8.55 (d, 1H), 8.03 (d, 2H), 7.85 (d, 1H), 7.70 (m, 3H), 7.63 (t, 1H), 7.48 (m, 2H), 7.09 (d, 1H), 5.23 (m, 1H), 4.81 (d, 2H), 4.03 (d, 1H), 3.74 (d, 1H), 3.08 (m, 2H), 2.20 (m, 1H), 0.97 (dd, 6H)

### Example 9: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 4-(trifluoromethyl)benzoate

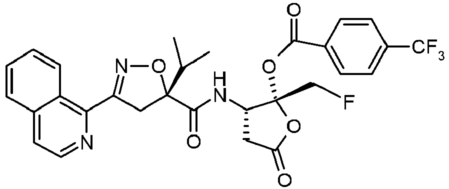

The compound of Formula 2 (0.5 g, 1.2 mmol) was dissolved in dichloromethane (20 mL), and then 4-trifluoromethyl benzoyl chloride (0.30 g, 1.4 mmol, 1.1 equiv) and 4-dimethylaminopyridine (0.29 g, 2.4 mmol, 2.0 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.1 g (yield: 13%) of the title compound.
¹H NMR (400 MHz, CDCl₃) δ 9.12 (d, 1H), 8.55 (d, 1H), 8.14 (d, 2H), 7.88 (d, 1H), 7.74 (m, 3H), 7.85 (m, 2H), 7.09 (d, 1H), 5.26 (m, 1H), 4.82 (d, 2H), 4.03 (d, 1H), 3.83 (d, 1H), 3.08 (m, 2H), 2.19 (m, 1H), 0.82 (dd, 6H)

### Example 10: (2S,3S)-2-(fluoromethyl)-3-((R)-5-isopropyl-3-(isoquinolin-1-yl)-4.,5-dihydroisoxazole-5-carboxamido)-5-oxotetrahydrofuran-2-yl 2-phenylacetate

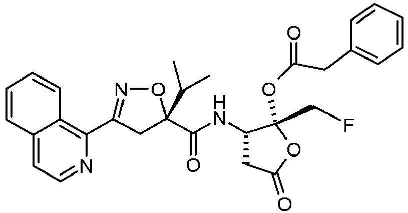

The compound of Formula 2 (0.5 g, 1.2 mmol) was dissolved in dichloromethane (20 mL), and then phenylacetyl chloride (0.22 g, 1.4 mmol, 1.1 equiv), triethylamine (0.18 g, 1.8 mmol, 1.5 equiv) and 4-dimethylaminopyridine (0.015 g, 0.12 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.3 g (yield: 51%) of the title compound.
¹H NMR (400 MHz, CDCl₃) δ 9.12 (d, 1H), 8.55 (d, 1H), 7.88 (d, 1H), 7.74-7.69 (m, 3H), 7.37 (m, 2H), 7.30(m, 3H), 7.09 (d, 1H), 5.23 (m, 1H), 4.69 (d, 2H), 4.03 (d, 1H), 3.83 (d, 1H), 3.74 (s, 2H), 2.95 (m, 2H), 2.38 (m, 1H), 1.05 (dd, 6H)

### Preparation Example 1: (S)-4,4-diethoxy-5-fluoro-3-((R)-5-isopropyl-3-(isoquinolin-1-yl)-4,5-dihydroisoxazole-5-carboxamido)pentanoic acid

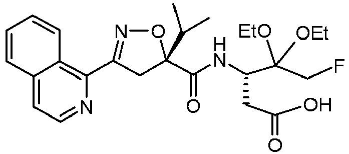

### (Step A) Ethyl (S)-5-fluoro-3-((R)-5-isopropyl-3-(isoquinolin-1-yl)-4,5-dihydroisoxazole-5-carboxamido)-4-oxopentanoate

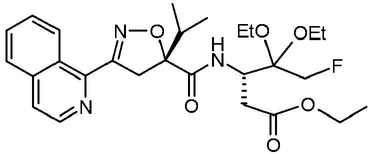

The compound of Formula 2 (500 mg, 1.2 mmol) was reacted with p-tosylic acid (114 mg, 0.6 mmol), triethoxymethane (20 ml, 120 mmol) and ethanol (20 ml) under reflux for 6 days. The reaction mixture was cooled to room temperature, saturated ammonium chloride solution was added thereto, and extracted with ethyl acetate. The organic layer was dried over sodium sulfate, concentrated and purified by the use of MPLC to obtain the title compound (230 mg, 37%).
¹H-NMR (CDCl₃) δ 9.15 (d, 1H), 8.54 (d, 1H), 7.84 (d, 1H), 7.75 ~ 7.64 (m, 3H), 4.75 ~ 4.86 (m, 1H) 4.53 (dd, 1H), 4.42 (dd, 1H), 4.02 (d, 1H) 3.97 ~ 3.67 (m, 5H), 3.57 ~ 3.50 (m, 3H), 2.71 (dd, 1H), 2.52 ~ 2.36 (m, 2H), 1.18 ∼ 0.97 (m, 15H)

### (Step B) (S)-4,4-diethoxy-5-fluoro-3-((R)-5-isopropyl-3-(isoquinolin-1-yl)-4,5-dihydroisoxazole-5-carboxamido)pentanoic acid

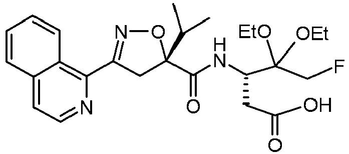

To ethyl (S)-5-fluoro-3-((R)-5-isopropyl-3-(isoquinolin-1-yl)-4,5-dihydroisooxazole-5-carboxamido)-4-oxopentanoate (230 mg, 0.44 mmol) and lithium hydroxide (31.9 mg, 1.33 mmol), water (1.12 ml) and tetrahydrofuran (THF, 0.28 ml) were added, and the reaction was carried out at 40°C for 3 hours. The reactant was cooled to room temperature, concentrated under reduced pressure, 1 N sodium hydroxide was added, and the water layer was washed with toluene. Then, 6N hydrochloric acid was added to adjust the pH to 3, followed by extraction using dichloromethane. The extracted organic layer was dried over sodium sulfate and filtered under reduced pressure to obtain the title compound. The obtained title compound was used in the next reaction without further purification.

### Example 11: (5R)-N-((3S)-2-ethoxy-2-(fluoromethyl)-5-oxotetrahydrofuran-3-yl)-5-isopropyl-3-(isoquinolin-1-yl)-4,5-dihydroisoxazole-5-carboxamide

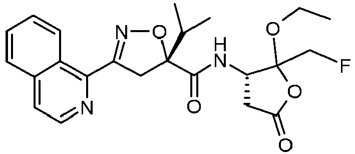

To (S)-4,4-diethoxy-5-fluoro-3-((R)-5-isopropyl-3-(isoquinolin-1-yl)-4,5-dihydroisooxazole-5-carboxamido)pentanoic acid (200 mg, 0.38 mmol), trifluoroacetic acid (0.5 ml) and dichloromethane (5 ml) were added at 0°C and and stirred at room temperature for 2 hours. After stirring for 2 hours, the reaction mixture was concentrated under reduced pressure and the title compound (92 mg, 54%) was obtained through MPLC.
¹H-NMR (CDCl₃) δ 9.11 (d, 1H), 8.56 (d, 1H), 7.86 (d, 1H), 7.75 ~ 7.64 (m, 3H), 7.38 (d, 1H) 4.94 (q, 1H) 4.66 (s, 1H), 4.54 (s, 1H), 4.09 (d, 1H), 4.02 (d, 1H) 3.90 ~ 3.67 (m, 3H), 2.92 (dd, 1H), 2.59 (dd, 1H), 2.36 (p, 1H), 1.29 ~ 1.19 (m, 4H), 1.06 (t, 6H)

### Preparation Example 2: (S)-5-fluoro-3-((R)-5-isopropyl-3-(isoquinolin-1-yl)-4,5-dihydroisoxazole-5-carboxamido)-4,4-dimethoxypentanoic acid

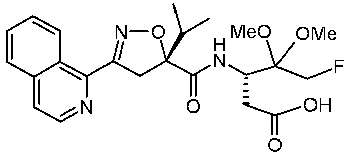

### (Step A) Methyl (S)-5-fluoro-3-((R)-5-isopropyl-3-(isoquinolin-1-yl)-4,5-dihydroisoxazole-5-carboxamido)-4,4-dimethoxypentanoate

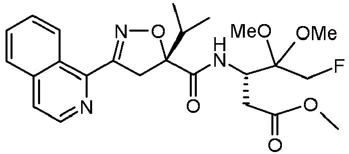

The compound of Formula 2 (1 g, 2.4 mmol) was reacted with p-tosylic acid (229 mg, 1.2 mmol), triethoxymethane (10 ml, 90 mmol) and methanol (20 ml) under reflux for 4 days. The reaction mixture was cooled to room temperature, saturated ammonium chloride solution was added thereto, and extracted with ethyl acetate. The organic layer was dried over sodium sulfate, concentrated and purified by the use of MPLC to obtain the title compound (561 mg, 49%).

### (Step B) (S)-5-fluoro-3-((R)-5-isopropyl-3-(isoquinolin-1-yl)-4,5-dihydroisoxazole-5-carboxamido)-4,4-dimethoxypentanoic acid

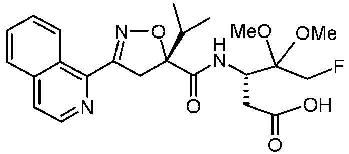

To methyl (S)-5-fluoro-3-((R)-5-isopropyl-3-(isoquinolin-1-yl)-4,5-dihydroisoxazole-5-carboxamido)-4,4-dimethoxypentanoate (562 mg, 1.18 mmol) and lithium hydroxide (134 mg, 5.59 mmol), water (3.42 ml) and THF (0.85 ml) were added, and the reaction was carried out at 40°C for 4 hours. The reactant was cooled to room temperature, concentrated under reduced pressure, 1 N sodium hydroxide was added, and the water layer was washed with toluene. Then, 6N hydrochloric acid was added to adjust the pH to 3, followed by extraction using dichloromethane. The extracted organic layer was dried over sodium sulfate and filtered under reduced pressure to obtain the title compound. The obtained title compound was used in the next reaction without further purification.

### Example 12: (5R)-N-((3S)-2-(fluoromethyl)-2-methoxy-5-oxotetrahydrofuran-3-yl)-5-isopropyl-3-(isoquinolin-1-yl)-4,5-dihydroisoxazole-5-carboxamide

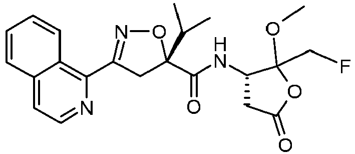

To (S)-5-fluoro-3-((R)-5-isopropyl-3-(isoquinolin-1-yl)-4,5-dihydroisoxazole-5-carboxamido)-4,4-dimethoxypentanoic acid (441 mg, 0.9 mmol), trifluoroacetic acid (0.5 ml) and dichloromethane (5 ml) were added at 0°C and and stirred at room temperature for 2 hours. After stirring for 2 hours, the mixture was concentrated under reduced pressure and the title compound (196 mg, 51%) was obtained through MPLC.
¹H-NMR (CDCl₃) δ 9.12 (d, 1H), 8.56 (d, 1H), 7.86 (d, 1H), 7.75 ~ 7.64 (m, 3H), 7.40 (d, 1H) 4.91 (q, 1H) 4.66 (dd, 1H), 4.54 (dd, 1H), 4.08 (d, 1H), 4.80 (d, 1H), 3.35 (s, 3H), 2.90 (dd, 1H), 2.56 (dd, 1H), 2.36 (p, 1H), 1.06 (t, 6H)

### Experimental Example 1: Hydrolysis test by hydrolase of prodrug compound of Formula 1

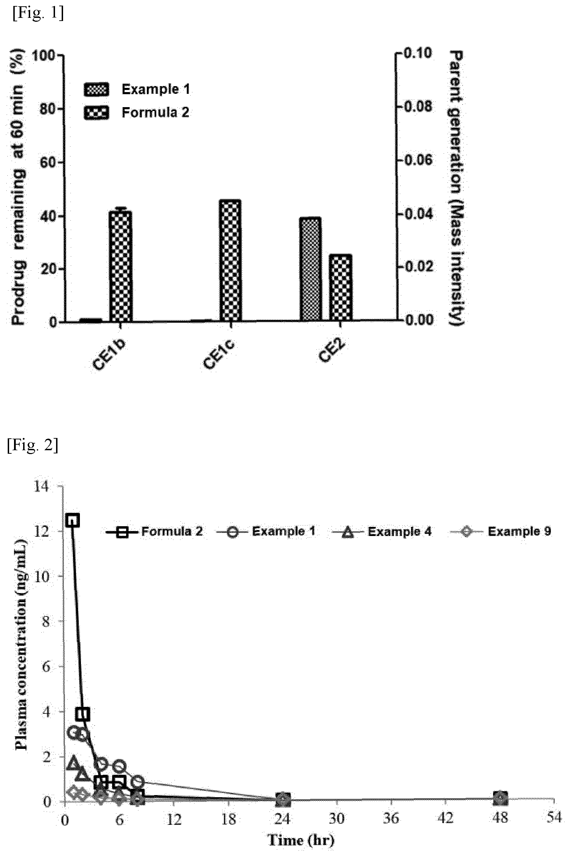

Various recombinant human carboxylesterases (CE1b, CE1c and CE2) were prepared in order to confirm the hydrolysis tendency of the prodrug by carboxylesterase (CE) which is a hydrolase. The initial enzyme concentration provided by the manufacturer was 5 mg/mL, which was diluted 1/50 in 100 mM potassium phosphate buffer to obtain a protein solution of 100 µg/mL. For the compound of Example 1-which is a prodrug compound to be tested, 5 mg/mL DMSO stock was used as a working solution, which was diluted 1/100 in acetonitrile to obtain 50 µg/mL concentration solution, and again diluted to a concentration of 1/100 in 100 mM potassium phosphate buffer to obtain a drug solution with a final concentration of 500 ng/mL. Then, each solution prepared above was mixed 1:1 to start the enzymatic reaction, and 50 µl of each sample was collected at 0 and 10 minutes. The samples were deproteinized with acetonitrile containing an internal standard (IS) and centrifuged, and the supernatant was injected into LC-MS/MS for analysis. By correcting the obtained peak area of the prodrug with the peak area of IS, the peak response at the time of collection of each sample was obtained, and the residual ratio (% remaining) compared to the initial value was converted. In addition, by correcting the obtained peak area of the compound of Formula 2 with the peak area of IS, the peak response at the time of collection of each sample was obtained, and generation according to time was confirmed (Figure 1).

As a prodrug of Formula 1, the compound of Example 1 was spiked with human recombinant CE isozyme and the residual ratio after reaction for 60 minutes was measured, confirming that most of the prodrug was lost. Such result is analyzed that the compound of Example 1 was hydrolyzed by an esterase present in the reaction system and converted into a compound of Formula 2, which is the parent drug. The contribution to these changes among each human recombinant CE isozyme was found to be greater by CE1b and CE1c than by CE2.

### Experimental Example 2: Pharmacokinetic test in mice

For subcutaneous injection (SC) PK testing of the compounds of Examples 1, 4 and 9 as a prodrug compound of Formula 1, about 7-week-old C57BL6 mice were prepared, and a group separation was carried out by allocating 3 animals per administration compound. Because it was a case of subcutaneous injection, fasting was not performed. On the day of administration, a drug solution was prepared at a concentration of 5 mg/mL by the use of 0.5% methyl cellulose (MC) as a vehicle, and this was injected subcutaneously in an amount of 10 mL per kg of body weight of each individual to make a final dose of 50 mg/kg. At 1, 2, 4, 6, 8, 24 and 48 hours after administration, blood was collected through the orbital vein, and the blood of each of the three animals per group was pooled in a heparin tube. The obtained blood sample was centrifuged at 15,000 rpm for 2 minutes to separate plasma, and 50 µl was taken and stored frozen at -20°C. On the day of the analysis, the sample was thawed at room temperature, and deproteinization was carried out with 200 µl of acetonitrile which was a total of 4 times the stored volume. At this time, acetonitrile contained an internal standard and 5% formic acid (FA). To prepare a calibration curve, acetonitrile solutions (including IS and 5% FA) with known concentrations of 0.1, 0.5, 5, 50 and 500 ng/mL, respectively, were prepared, and the blank serum was deproteinized with 4 times the volume of acetonitrile as above to prepare a calibration curve of final 0.4 - 2,000 ng/mL. After injecting 0.5 µl of the supernatant obtained after deproteinization to LC-MS/MS, the peak area of the compound of Formula 2 was corrected to the IS peak area to obtain the peak response at each sample collection point, and the concentration was converted through a calibration curve. Pharmacokinetic parameters (Cₘₐₓ, Tₘₐₓ, AUCₗₐₛₜ, tvz, etc.) were calculated through a noncompartmental analysis method using WinNonlin 8.1 for the value of blood concentration according to time for each administration group. The pharmacokinetic characteristics of each compound were compared by comparing exposure and half-life changes by the drug administration group.

In the case of subcutaneous injection of a prodrug of the compound of Formula 2-which is the parent drug, the peak blood drug concentration value of the compound of Formula 2 was decreased (Figure 2). The characteristics observed in the case of administering the prodrug are advantageous in terms of the expression of side effects caused by the high blood concentration because there is a small difference in drug concentration. In addition, it is expected to have advantages over direct administration of the parent drug to maintain the effective concentration required for the expression of efficacy and achieve the desired efficacy.

The pharmacokinetic parameters of the parent drug-which is a metabolite measured in the plasma of mice after subcutaneous administration of the compounds of Examples 1, 4 and 9, which are the prodrug compounds of Formula 1-are represented in Table 1 below.

**[Table 1]**

| | Formula 2 | Example 1 | Example 4 | Example 9 |
|---|---|---|---|---|
| | Mean | Mean | Mean | Mean |
| Cₘₐₓ (µg/mL) | 12.50 | 3.08 | 1.70 | 0.41 |
| Tₘₐₓ (hr) | 1 | 1 | 1 | 1 |
| AUCₗₐₛₜ (hr×µg/mL) | 24.02 | 22.44 | 7.11 | 2.72 |

## Claims

1. A compound of the following Formula 1, or a pharmaceutically acceptable salt or isomer thereof: 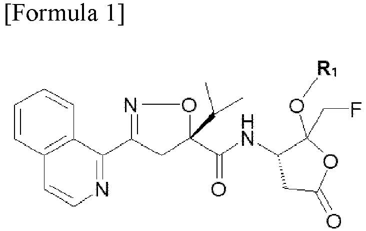 wherein
R₁ represents alkyl, cycloalkyl, aryl or -C(O)R₂;
R₂ represents alkyl, cycloalkyl, aryl, arylalkyl, or heteroaryl including one or more heteroatoms selected from N, O and S; and
the alkyl, cycloalkyl, arylalkyl and heteroaryl are optionally substituted, and the substituent may be one or more selected from alkyl, cycloalkyl, hydroxy, halo, haloalkyl, acyl, amino, alkoxy, carboalkoxy, carboxy, carboxyamino, cyano, nitro, thiol, aryloxy, sulfoxy and guanido group.

2. The compound, or a pharmaceutically acceptable salt or isomer thereof according to Claim 1, wherein
R₁ represents Ci-Cs alkyl or -C(O)R₂; and
R₂ represents C₁-C₂₀ alkyl, C₆-C₁₀ aryl or C₆-C₁₀ aryl-C₁-C₇ alkyl.

3. The compound, or a pharmaceutically acceptable salt or isomer thereof according to Claim 1, wherein
R₁ represents C₁-C₅ alkyl or -C(O)R₂;
R₂ represents C₁-C₁₅ alkyl, C₆-C₁₀ aryl or C₆-C₁₀ aryl-C₁-C₅ alkyl; and
the substituent is alkyl or haloalkyl.

4. The compound according to Claim 1, wherein the compound is selected from the following group:
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl acetate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl propionate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl isobutyrate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl pivalate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 3-methylbutanoate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 3,3-dimethylbutanoate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl palmitate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl benzoate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 4-(trifluoromethyl)benzoate;
(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 2-phenylacetate;
(5R)-N-((3S)-2-ethoxy-2-(fluoromethyl)-5-oxotetrahydrofuran-3-yl)-5-isopropyl-3-(isoquinolin-1-yl)-4,5-dihydroisoxazole-5-carboxamide; and
(5R)-N-((3S)-2-(fluoromethyl)-2-methoxy-5-oxotetrahydrofuran-3-yl)-5-isopropyl-3-(isoquinolin-1-yl)-4,5-dihydroisoxazole-5-carboxamide.

5. A pharmaceutical composition for use in the prevention or treatment of inflammation or apoptosis comprising the compound of Formula 1, or a pharmaceutically acceptable salt or isomer thereof as defined in any one of Claims 1 to 4 as an active ingredient, together with a pharmaceutically acceptable carrier.

6. The pharmaceutical composition for use according to Claim 5, which is formulated in oral dosage form, injection form or patch form.

7. A pharmaceutical composition for use in the prevention or treatment of a disease selected from apoptosis-associated diseases, inflammatory diseases, osteoarthritis, rheumatoid arthritis, degenerative arthritis and destructive bone disorders, comprising the compound of Formula 1, or a pharmaceutically acceptable salt or isomer thereof as defined in any one of Claims 1 to 4 as an active ingredient, together with a pharmaceutically acceptable carrier.

## Patentansprüche

1. Verbindung der folgenden Formel 1 oder ein pharmazeutisch annehmbares Salz oder Isomer davon: 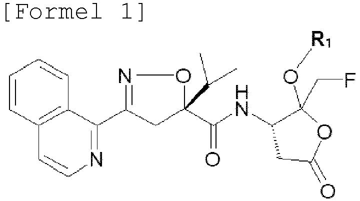 worin
R₁ Alkyl, Cycloalkyl, Aryl oder -C(O)R₂ darstellt;
R₂ Alkyl, Cycloalkyl, Aryl, Arylalkyl oder Heteroaryl, das ein oder mehrere Heteroatome, ausgewählt aus N, O und S, enthält, darstellt; und
das Alkyl, Cycloalkyl, Arylalkyl und Heteroaryl gegebenenfalls substituiert sind und der Substituent einer oder mehrere sein kann, ausgewählt aus Alkyl-, Cycloalkyl-, Hydroxy-, Halo-, Haloalkyl-, Acyl-, Amino-, Alkoxy-, Carboalkoxy-, Carboxy-, Carboxyamino-, Cyano-, Nitro-, Thiol-, Aryloxy-, Sulfoxy- und Guanidogruppe.

2. Verbindung oder ein pharmazeutisch annehmbares Salz oder Isomer davon gemäß Anspruch 1, worin
R₁ C₁-C₈-Alkyl oder -C(O)R₂ darstellt; und
R₂ C₁-C₂₀-Alkyl, C₆-C₁₀-Aryl oder C₆-C₁₀-Aryl-C₁-C₇-alkyl darstellt.

3. Verbindung oder ein pharmazeutisch annehmbares Salz oder Isomer davon gemäß Anspruch 1, worin
R₁ C₁-C₅-Alkyl oder -C(O)R₂ darstellt;
R₂ C₁-C₁₅-Alkyl, C₆-C₁₀-Aryl oder C₆-C₁₀-Aryl-C₁-C₅-alkyl darstellt; und
der Substituent Alkyl oder Haloalkyl ist.

4. Verbindung gemäß Anspruch 1, worin die Verbindung aus der folgenden Gruppe ausgewählt ist:
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl-acetat;
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl-propionat;
(2R,3S)-2-(Fluormethyl)-3-((R)-5-isopropyl-3-(isoquinolin-1-yl)-4,5-dihydroisoxazol-5-carboxamido)-5-oxotetrahydrofuran-2-yl-isobutyrat;
(2R,3S)-2-(Fluormethyl)-3-((R)-5-isopropyl-3-(isoquinolin-1-yl)-4,5-dihydroisoxazol-5-carboxamido)-5-oxotetrahydrofuran-2-yl-pivalat;
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl-3-methylbutanoat;
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl-3,3-dimethylbutanoat;
(2S,3S)-2-(Fluormethyl)-3-((R)-5-isopropyl-3-(isoquinolin-1-yl)-4,5-dihydroisoxazole-5-carboxamido)-5-oxotetrahydrofuran-2-yl-palmitat;
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl-benzoat;
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl 4-(trifluormethyl)benzoat;
(2S,3S)-2-(Fluormethyl)-3-((R)-5-isopropyl-3-(isoquinolin-1-yl)-4,5-dihydroisoxazol-5-carboxamido)-5-oxotetrahydrofuran-2-yl 2-phenylacetat;
(5R)-N-((3S)-2-Ethoxy-2-(fluormethyl)-5-oxotetrahydrofuran-3-yl)-5-isopropyl-3-(isoquinolin-1-yl)-4,5-dihydroisoxazol-5-carboxamid; und
(5R)-N-((3S)-2-(Fluormethyl)-2-methoxy-5-oxotetrahydrofuran-3-yl)-5-isopropyl-3-(isoquinolin-1-yl)-4,5-dihydroisoxazol-5-carboxamid.

5. Pharmazeutische Zusammensetzung zur Verwendung bei der Vorbeugung oder Behandlung von Entzündung oder Apoptose, umfassend die Verbindung der Formel 1 oder ein pharmazeutisch annehmbares Salz oder Isomer davon wie in einem der Ansprüche 1 bis 4 definiert als Wirkstoff, zusammen mit einem pharmazeutisch annehmbaren Träger.

6. Pharmazeutische Zusammensetzung zur Verwendung gemäß Anspruch 5, die in oraler Darreichungsform, Injektionsform oder Patchform formuliert ist.

7. Pharmazeutische Zusammensetzung zur Verwendung bei der Vorbeugung oder Behandlung einer Erkrankung, ausgewählt aus Apoptose-assoziierten Erkrankungen, entzündlichen Erkrankungen, Osteoarthritis, rheumatoider Arthritis, degenerativer Arthritis und destruktiver Knochenerkrankungen, umfassend die Verbindung der Formel 1 oder ein pharmazeutisch annehmbares Salz oder Isomer davon wie in einem der Ansprüche 1 bis 4 definiert als Wirkstoff, zusammen mit einem pharmazeutisch annehmbaren Träger.

## Revendications

1. Composé selon la Formule 1 suivante, ou sel ou isomère de celui-ci pharmaceutiquement acceptable : 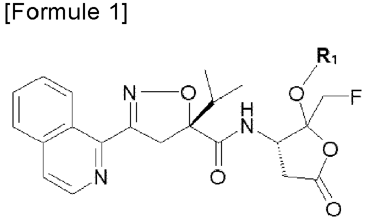 dans lequel
R₁ représente un alkyle, un cycloalkyle, un aryle ou -C(O)R₂ ;
R₂ représente un alkyle, un cycloalkyle, un aryle, un arylalkyle ou un hétéroaryle incluant un ou plusieurs hétéroatomes choisis parmi N, O et S ; et
l'alkyle, le cycloalkyle, l'arylalkyle et l'hétéroaryle sont facultativement substitués, et le substituant peut être un ou plusieurs choisis parmi un groupe alkyle, cycloalkyle, hydroxy, halogéno, halogénoalkyle, acyle, amino, alcoxy, carboalcoxy, carboxy, carboxyamino, cyano, nitro, thiol, aryloxy, sulfoxy et guanido.

2. Composé, ou sel ou isomère de celui-ci pharmaceutiquement acceptable selon la revendication 1, dans lequel
R₁ représente un alkyle en C₁-C₈ ou -C(O)R₂ ; et
R₂ représente un alkyle en C₁-C₂₀, un aryle en C₆-C₁₀ ou un aryl en C₆-C₁₀-alkyle en C₁-C₇.

3. Composé, ou sel ou isomère de celui-ci pharmaceutiquement acceptable selon la revendication 1, dans lequel
R₁ représente un alkyle en C₁-C₅ ou -C(O)R₂ ;
R₂ représente un alkyle en C₁-C₁₅, un aryle en C₆-C₁₀ ou un aryle en C₆-C₁₀-alkyle en C₁-C₅ ; et
le substituant est un alkyle ou un halogénoalkyle.

4. Composé selon la revendication 1, dans lequel le composé est choisi dans le groupe suivant :
acétate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
propionate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
isobutyrate de (2R,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
pivalate de (2R,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
3-méthylbutanoate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
3,3-diméthylbutanoate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
palmitate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
benzoate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
4-(trifluorométhyl)benzoate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
2-phénylacétate de (2S,3S)-2-(fluorométhyl)-3-((R)-5-isopropyl-3-(isoquinolin-1-yl)-4,5-dihydroisoxazole-5-carboxamido)-5-oxotétrahydrofuran-2-yle ;
(5R)-N-((3S)-2-éthoxy-2-(fluorométhyl)-5-oxotétrahydrofuran-3-yl)-5-isopropyl-3-(isoquinolin-1-yl)-4,5-dihydroisoxazole-5-carboxamide ; et
(5R)-N-((3S)-2-(fluorométhyl)-2-méthoxy-5-oxotétrahydrofuran-3-yl)-5-isopropyl-3-(isoquinolin-1-yl)-4,5-dihydroisoxazole-5-carboxamide.

5. Composition pharmaceutique pour utilisation dans la prévention ou le traitement d'une inflammation ou d'une l'apoptose comprenant le composé de Formule 1, ou un sel ou isomère de celui-ci pharmaceutiquement acceptable selon l'une quelconque des revendications 1 à 4 en tant que principe actif, conjointement avec un vecteur pharmaceutiquement acceptable.

6. Composition pharmaceutique pour une utilisation selon la revendication 5, qui est formulée sous une forme posologique orale, une forme d'injection ou une forme de timbre.

7. Composition pharmaceutique pour utilisation dans la prévention ou le traitement d'une maladie choisie parmi les maladies associées à l'apoptose, les maladies inflammatoires, l'arthrose, la polyarthrite rhumatoïde, l'arthrite dégénérative et les troubles osseux destructifs, comprenant le composé de Formule 1, ou un sel ou isomère de celui-ci pharmaceutiquement acceptable selon l'une quelconque des revendications 1 à 4 en tant que principe actif, conjointement avec un vecteur pharmaceutiquement acceptable.
